# EUROPEAN PATENT APPLICATION

(11) **EP 4 450 622 A1**
(43) Date of publication of application: **23.10.2024**
(21) Application number: 22907470.3
(22) Date of filing: 14.12.2022
(51) Int. Cl.: C12N 15/11, C12Q 1/6839, C12Q 1/6851, C12Q 1/6883, G01N 33/50

(54) **DATA COLLECTION METHOD AND KIT FOR DETERMINING LIKELIHOOD OF DEVELOPING ALZHEIMER'S DISEASE**

(30) Priority: 17.12.2021 JP 2021205192
(71) Applicant: Kobayashi, Nobuyuki, Tokyo, 105-8461 (JP)
(72) Inventor: Kobayashi, Nobuyuki, Tokyo, 105-8461 (JP)
(74) Representative: EIP
(86) International application number: PCT/JP2022/045985
(87) International publication number: WO 2023/112946

(57) **Abstract**

A data collection method for determining the likelihood that a subject will develop AD that includes measurement of methylation levels in the CpG regions of promoter regions, untranslated regions or translated regions of specific genes in biological samples from subjects, wherein higher or lower methylation levels compared with NCs indicate that a subject is at risk of developing AD.

## Description

### TECHNICAL FIELD

This invention concerns a data collection method and kit for determining the likelihood of developing Alzheimer's disease.

This application claims priority based on patent No. 2021-205192 filed on December 17 2021 in Japan and its content is referenced herein.

### BACKGROUND ART

Alzheimer's disease (AD) is a common type of dementia. Although AD is considered to be due to the deposition of Amyloid β and phosphorylated tau in brain tissue, its pathogenesis mechanism remains unknown. Owing to the involvement in epigenetics of genetic factors, aging and environmental factors, such as lifestyle habits and infections, these factors are thought to be involved in AD pathogenesis.

Consultations with specialists are conducted for early discovery of AD but a precise diagnosis cannot be made before the disease develops and making one is difficult. Testing for amyloid β in spinal fluid and positron emission tomography (PET) are commonly used examination methods. However, the former is highly invasive, PET diagnosis is costly, only a limited number of facilities can conduct these examinations and they are not covered by health insurance, making them impractical clinically.

No blood marker for the diagnosis of AD is currently available in clinical practice. The present inventors comprehensively quantified changes in methylation levels in blood DNA in healthy individuals (NCs), patients with AD and patients with amnesic mild cognitive impairment (aMCI), an earlier stage of AD, and discovered that DNA methylation was significantly reduced in the NCAPH2 (LMF2) gene promotor region in patients with AD and aMCI, and also that DNA methylation was significantly elevated in the COASY gene and SPINT-1 gene promoter regions. They previously reported that these biomarkers could potentially be useful in the early diagnosis of dementia and differentiation of dementia types (Non-patent references 1-3)

### Prior art references

### Non-patent references

[Non-patent reference 1] Kobayashi N, Shinagawa S, Nagata T, Shimada K, Shibata N, Ohnuma T, Kasanuki K, Arai H, Yamada H, Nakayama K, Kondo K. Development of Biomarkers Based on DNA Methylation in the NCAPH2/LMF2 Promoter Region for Diagnosis of Alzheimer's Disease and Amnesic Mild Cognitive Impairment. PLoS One 2016; 11(1): e0146449.
[Non-patent reference 2] Kobayashi N, Shinagawa S, Nagata T, Shimada K, Shibata N, Ohnuma T, Kasanuki K, Arai H, Yamada H, Nakayama K, Kondo K. Usefulness of DNA Methylation Levels in COASY and SPINT1 Gene Promoter Regions as Biomarkers in Diagnosis of Alzheimer's Disease and Amnestic Mild Cognitive Impairment. PLoS One 2016;11(12): e0168816.
[Non-patent reference 3] Kobayashi N, Shinagawa S, Niimura H, Kida H, Nagata T, Tagai K, Shimada K, Oka N, Shikimoto R, Noda Y, Nakajima S, Mimura M, Shigeta M, Kondo K. Increased blood COASY DNA methylation levels a potential biomarker for early pathology of Alzheimer's disease. Sci Rep 2020; 10(1): 12217.

### SUMMARY OF INVENTION

### Technical Problem

Against this background, knowing that aging is a major risk factor for AD, the present inventors considered that DNA methylation levels could be a useful biomarker in the diagnosis of AD because they change significantly with age. They identified regions with changes in methylation levels with age as well as those with significant differences in DNA methylation levels between elderly NCs and AD patients and aMCI patients, finding that regions in which methylation levels changed with age were common to those with significant differences in methylation levels between NCs and AD and aMC1 patients. To complete the present invention, they discovered that DNA methylation levels could potentially be useful in determining the likelihood of developing AD. Thus, the present invention aims to provide useful technology for determining the risk of developing AD by measuring the percentage of DNA methylation in novel genes.

### Solution to Problem

The present invention includes the following aspects.
[1] The present invention comprises a data collection method for determining the likelihood that a subject will develop AD that includes measuring the percentage of DNA methylation in biological samples from subjects in the CpG regions of promoter regions, untranslated regions or translated regions of at least one gene selected from groups consisting of the following, wherein higher or lower methylation levels compared with NCs indicate that a subject is at risk of developing AD: ELOVL2, OLIG3, SLC7A14, ACTA1, SOX17, DPYS, LOC728661, CDK11B, NEFM, SMTN, GDF15, RUNX3, PPP1R14A, ZNF75A, C4orf41, RWDD4A, ADAM12, PENK, TBX15, GREM1, KIAA0895L, EXOC3L, TM6SF2, CSNK1G2, FAM53A, DOCK1, FAM196A, WDR81, TCEA2, C3orf26, FILIP1L, MIR548G, CCDC28A, NPY1R, KIAA0182, MARCH1, XPNPEP3, ST13, FAM5B, TCP11, SLC25A2, ZNF415, PCDH8, TIMM44, CTXN1, HIST1H3D, HIST1H2BF, HIST1H2AD, RBMXL3, LRCH2, LOXL1, SLC34A2, RASGRP2, PCDHB7, SLC12A8, KANK1, ALPK3, FARP1, WDR17, GNAS, GNASAS, SOLH, ATL2, PCDHB 10, PDE4C, NKAIN3, GABRA2, PCDHA2, PCDHA1, PCDHA3, PCDHA4, PCDHA5, FZD9, ATP5SL, PCDHB4, PRR23A, ADC, ABCA1, L3MBTL4, TSSK6, PCDHA6, ANKRD45, FAM163A, ESRRA, PEAR1, IRS2, GFI1, KCTD1, FGR, LOC100130691, AGPS, CSNK1G1, APAF1, IKBIP, PRIC285, ZBTB7B, TEF, EGLN2, LMBR1L, C19orf39, MEA1, KLHDC3, RHPN1, C8orf51, FLCN, PQLC1, TRIM23, C5orf44, PRR3, GNL1, PYCR1, SPTBN4, BLURB, BAZ1A, ZFHX3, SLC25A46, RBM14, HNRNPC, HDAC11, SLC16A14, C4orf33, SCLT1, GPR68, HSPA8, ZSWIM7, TTC19, PRPF40B, RAB10, SLC37A1, AMD1, ANKLE2, CD55, SSBP4, MGC23284, SNAI3, SMG1, APBB1IP, NDUFB1, CPSF2, MBTD1, UTP18, NPBWR2, RWDD2A, PGM3, MZF1, LOC100131691, C19orf12, LIN54, PHLDA2, SERINC5, MPV17L2, ZNF10, C3orf39, GPX1, TRPC4AP, REST and TBKBP1.
[2] Data collection procedure described in [1], wherein biological samples are blood
[3] Kit for determining the likelihood that subjects will develop AD that includes primers or probes for the amplification of DNA methylation regions in CpG regions of gene promoter regions, untranslated regions or translated regions of at least one gene selected from groups consisting of the following: ELOVL2, OLIG3, SLC7A14, ACTA1, SOX17, DPYS, LOC728661, CDK11B, NEFM, SMTN, GDF15, RUNX3, PPP1R14A, ZNF75A, C4orf41, RWDD4A, ADAM12, PENK, TBX15, GREM1, KIAA0895L, EXOC3L, TM6SF2, CSNK1G2, FAM53A, DOCK1, FAM196A, WDR81, TCEA2, C3orf26, FILIP1L, MIR548G, CCDC28A, NPY1R, KIAA0182, MARCH1, XPNPEP3, ST13, FAM5B, TCP11, SLC25A2, ZNF415, PCDH8, TIMM44, CTXN1, HIST1H3D, HIST1H2BF, HIST1H2AD, RBMXL3, LRCH2, LOXL1, SLC34A2, RASGRP2, PCDHB7, SLC12A8, KANK1, ALPK3, FARP1, WDR17, GNAS, GNASAS, SOLH, ATL2, PCDHB10, PDE4C, NKAIN3, GABRA2, PCDHA2, PCDHA1, PCDHA3, PCDHA4, PCDHA5, FZD9, ATP5SL, PCDHB4, PRR23A, ADC, ABCA1, L3MBTL4, TSSK6, PCDHA6, ANKRD45, FAM163A, ESRRA, PEAR1, IRS2, GFI1, KCTD1, FGR, LOC100130691, AGPS, CSNK1G1, APAF1, IKBIP, PRIC285, ZBTB7B, TEF, EGLN2, LMBR1L, C19orf39, MEA1, KLHDC3, RHPN1, C8orf51, FLCN, PQLC1, TRIM23, C5orf44, PRR3, GNL1, PYCR1, SPTBN4, BLURB, BAZ1A, ZFHX3, SLC25A46, RBM14, HNRNPC, HDAC11, SLC16A14, C4orf33, SCLT1, GPR68, HSPA8, ZSWIM7, TTC19, PRPF40B, RAB10, SLC37A1, AMD1, ANKLE2, CD55, SSBP4, MGC23284, SNAI3, SMG1, APBB1IP, NDUFB1, CPSF2, MBTD1, UTP18, NPBWR2, RWDD2A, PGM3, MZF1, LOC100131691, C19orf12, LIN54, PHLDA2, SERINC5, MPV17L2, ZNF10, C3orf39, GPX1, TRPC4AP, REST and TBKBP1

### Advantageous Effects of Invention

The present invention provides useful technology for determining the likelihood that a subject will develop AD.

### BRIEF DESCRIPTION OF DRAWINGS

Figure 1 is a graph showing the percentage of DNA methylation in the CpG islands of the individual genes ELOVL2, OLIG3, SLC7A14, ACTA1, SOX17, DPYS, LOC728661, and CDK11B in elderly NCs, AD patients and aMCI patients.
Figure 2 is a graph showing the percentage of DNA methylation in the CpG islands of the individual genes NEFM, SMTN, GDF15, RUNX3, PPP1R14A, ZNF75A, C4orf41, RWDD4A, and ADAM12 in elderly NCs, AD patients and aMCI patients.
Figure 3 is a graph showing the percentage of DNA methylation in the CpG islands of the individual genes PENK, TBX15, GREM1, KIAA0895L, EXOC3L, TM6SF2, CSNK1G2, FAM53A, DOCK1, and FAM196Ain elderly NCs, AD patients and aMCI patients.
Figure 4 is a graph showing the percentage of DNA methylation in the CpG islands of the individual genes WDR81, TCEA2, C3orf26, FILIP1L, MIR548G, CCDC28A, NPY1R, KIAA0182, MARCH1, XPNPEP3, and ST13 in elderly NCs, AD patients and aMCI patients.
Figure 5 is a graph showing the percentage of DNA methylation in the CpG islands of the individual genes FAM5B, TCP11, SLC25A2, ZNF415, PCDH8, TIMM44, CTXN1, HIST1H3D, HIST1H2BF, HIST1H2AD, RBMXL3, and LRCH2 in elderly NCs, AD patients and aMCI patients.
Figure 6 is a graph showing the percentage of DNA methylation in the CpG islands of the individual genes LOXL1, SLC34A2, RASGRP2, PCDHB7, SLC12A8, KANK1, and ALPK3 in elderly NCs, AD patients and aMCI patients.
Figure 7 is a graph showing the percentage of DNA methylation in the CpG islands of the individual genes FARP1, WDR17, GNAS, GNASAS, SOLH, ATL2, PCDHB10, and PDE4C in elderly NCs, AD patients and aMCI patients.
Figure 8 is a graph showing the percentage of DNA methylation in the CpG islands of the individual genes NKAIN3, GABRA2, PCDHA1, PCDHA2, PCDHA3, PCDHA4, PCDHA5, FZD9, ATP5SL, PCDHB4, PRR23A, and ADC in elderly NCs, AD patients and aMCI patients.
Figure 9 is a graph showing the percentage of DNA methylation in the CpG islands of the individual genes ABCA1, L3MBTL4, TSSK6, PCDHA1, PCDHA2, PCDHA3, PCDHA4, PCDHA5, PCDHA6, ANKRD45, FAM163AESRRA, and PEAR1 in elderly NCs, AD patients and aMCI patients.
Figure 10 is a graph showing the percentage of DNA methylation in the CpG islands of the individual genes IRS2, GFI1, KCTD1, PCDHA1, PCDHA2, PCDHA3, PCDHA4, PCDHA5, FGR, LOC100130691, AGPS, CSNK1G1, APAF1, and IKBIP in elderly NCs, AD patients and aMCI patients.
Figure 11 is a graph showing the percentage of DNA methylation in the CpG islands of the individual genes PRIC285, ZBTB7B, TEF, EGLN2, LMBR1L, C19orf39, MEA1, KLHDC3, RHPN1, and C8orf51 in elderly NCs, AD patients and aMCI patients.
Figure 12 is a graph showing the percentage of DNA methylation in the CpG islands of the individual genes FLCN, PQLC1, TRIM23, C5orf44, PRR3, GNL1, PYCR1, SPTBN4, BLURB, BAZ1A, and ZFHX3 in elderly NCs, AD patients and aMCI patients.
Figure 13 is a graph showing the percentage of DNA methylation in the CpG islands of the individual genes SLC25A46, RBM14, HNRNPC, HDAC11, SLC16A14, C4orf33, SCLT1, GPR68, and HSPA8 in elderly NCs, AD patients and aMCI patients.
Figure 14 is a graph showing the percentage of DNA methylation in the CpG islands of the individual genes ZSWIM7, TTC19, PRPF40B, RAB10, SLC37A1, AMD1, ANKLE2, CD55, and SSBP4 in elderly NCs, AD patients and aMCI patients.
Figure 15 is a graph showing the percentage of DNA methylation in the CpG islands of the individual genes GC23284, SNAI3, SMG1, BAZ1A, APBB1IP, NDUFB1, CPSF2, MBTD1, UTP18, NPBWR2, RWDD2A, and PGM3 in elderly NCs, AD patients and aMCI patients.
Figure 16 is a graph showing the percentage of DNA methylation in the CpG islands of the individual genes MZF1, LOC100131691, C19orf12, LIN54, PHLDA2, SERINC5, MPV17L2, ZNF10, and C3orf39 in elderly NCs, AD patients and aMCI patients.
Figure 17 is a graph showing the percentage of DNA methylation in the CpG islands of the individual genes GPX1, TRPC4AP, REST, and TBKBP1 in elderly NCs, AD patients and aMCI patients.

### DESCRIPTION OF EMBODIMENTS

### [Data collection method]

In one embodiment, the present invention provides a data collection method for determining the likelihood that a subject will develop AD that includes measuring the percentage of DNA methylation in biological samples from subjects in CpG regions of promoter regions, untranslated regions or translated regions of at least one gene selected from groups consisting of the following, wherein higher or lower methylation levels compared with NCs indicate that a subject is at risk of developing AD: ELOVL2, OLIG3, SLC7A14, ACTA1, SOX17, DPYS, LOC728661, CDK11B, NEFM, SMTN, GDF15, RUNX3, PPP1R14A, ZNF75A, C4orf41, RWDD4A, ADAM12, PENK, TBX15, GREM1, KIAA0895L, EXOC3L, TM6SF2, CSNK1G2, FAM53A, DOCK1, FAM196A, WDR81, TCEA2, C3orf26, FILIP1L, MIR548G, CCDC28A, NPY1R, KIAA0182, MARCH1, XPNPEP3, ST13, FAM5B, TCP11, SLC25A2, ZNF415, PCDH8, TIMM44, CTXN1, HIST1H3D, HIST1H2BF, HIST1H2AD, RBMXL3, LRCH2, LOXL1, SLC34A2, RASGRP2, PCDHB7, SLC12A8, KANK1, ALPK3, FARP1, WDR17,

### GNAS, GNASAS, SOLH, ATL2, PCDHB 10, PDE4C, NKAIN3, GABRA2, PCDHA2,

PCDHA1, PCDHA3, PCDHA4, PCDHA5, FZD9, ATP5SL, PCDHB4, PRR23A, ADC, ABCA1, L3MBTL4, TSSK6, PCDHA6, ANKRD45, FAM163A, ESRRA, PEAR1, IRS2, GFI1, KCTD1, FGR, LOC100130691, AGPS, CSNK1G1, APAF1, IKBIP, PRIC285, ZBTB7B, TEF, EGLN2, LMBR1L, C19orf39, MEA1, KLHDC3, RHPN1, C8orf51, FLCN, PQLC1, TRIM23, C5orf44, PRR3, GNL1, PYCR1, SPTBN4, BLURB, BAZ1A, ZFHX3, SLC25A46, RBM14, HNRNPC, HDAC11, SLC16A14, C4orf33, SCLT1, GPR68, HSPA8, ZSWIM7, TTC19, PRPF40B, RAB10, SLC37A1, AMD1, ANKLE2, CD55, SSBP4, MGC23284, SNAI3, SMG1, APBB1IP, NDUFB1, CPSF2, MBTD1, UTP18, NPBWR2, RWDD2A, PGM3, MZF1, LOC100131691, C19orf12, LIN54, PHLDA2, SERINC5, MPV17L2, ZNF10, C3orf39, GPX1, TRPC4AP, REST and TBKBP1.

As described later in the working example, the present embodiment allows data to be collected for the purpose of determining the likelihood that subjects will develop AD. However, the data collection method does not include a process for a doctor to make such a determination. The method of this embodiment may also be described as a procedure for determining whether there is a possibility of a subject developing AD.

In the method of the present embodiment, the percentage of DNA methylation in CpG regions of promoter regions, untranslated or translated regions of the genes mentioned above is the data for determining whether a subject is at risk of developing Alzheimer's disease.

Also, in the method of the present embodiment, the control is the percentage of DNA methylation in CpG regions of the promoter regions, untranslated regions or translated regions of these genes in biological samples from subjects in whom it has been previously established that Alzheimer's disease has not developed. Such subjects include healthy individuals and healthy elderly individuals.

The method of the present embodiment involves measuring the percentage of DNA methylation in the CpG regions of promoter regions, untranslated regions or translated regions of the genes mentioned above in biological samples from subjects. If the percentage of DNA methylation in the CpG regions of promoter regions, untranslated or translated regions of these genes in biological samples from subjects is higher or lower compared to NCs it can be determined that they are more likely to develop Alzheimer's disease.

In the method of this embodiment, there are no particular restrictions on biological samples as long they provide DNA data for the purpose of determining Alzheimer's disease risk. They could be blood, hair, saliva or hair but from the aspects of low invasiveness and abundant DNA content, blood is preferable. Specifically, genome DNA present in biological samples is extracted to measure the percentage DNA methylation in CpG regions of the promoter regions, untranslated regions or translated regions of the genes mentioned above.

CpG region means a location in DNA where there are phosphodiester bonds between cytosine and guanine. Regions where CpG regions appear at high frequency are called CpG islands. The "p" in CpG indicates a phosphodiester bond between cytosine and guanine. In the method of the present embodiment, CpG islands or CpG regions in their vicinity are preferable. CpG regions may be those in promoter regions, untranslated or translated regions of the genes mentioned above. However, since many mammalian genes have CpG regions within their promoter regions or in their vicinity, CpG regions should preferably be those in promoter regions and CpG islands in promoter regions are particularly desirable.

Tables 1-5 show the CpG site IDs, Reference gene names, RefSeq mRNA accession numbers, UCSC Genome Browser gene categories and CpG island locations used in the present embodiment. In Tables 1-5, genes with the same name have a separate listing if methylation locations and assigned CpG site IDs differ. Also, genes with different names are listed in the same row if methylation sites are the same and assigned CpG site IDs are the same.

**[Table 1]**

| CpG site ID | Reference gene name | RefSeq mRNA accession number | UCSC Genome Browser gene category | CpG island location |
|---|---|---|---|---|
| cg16867657 | ELOVL2 | NM_017770 | TSS1500 | chr6:11043913-11045206 |
| cg21572722 | ELOVL2 | NM_017770 | TSS1500 | chr6:11043913-11045206 |
| cg01889237 | OLIG3 | NM_175747 | TSS1500 | chr6:137816474-137817223 |
| cg07766263 | SLC7A14 | NM_020949 | 5'UTR | chr3:170303044-170303249 |
| cg14852384 | ACTA1 | NM_001100 | TSS200 | chr1:229567206-229570684 |
| cg02919422 | SOX17;SOX17 | NM_022454;NM_022454 | 5'UTR:1 stExon | chrB:55370170-55372525 |
| cg14015441 | DPYS | NM_001385 | TSS200 | chr8:105478672-105479340 |
| cg11530028 | LOC728661;CDK11B;CDK11B;CD K11B;CDK11B;CDK11B;CDK11B | NM_0011_10781;NM_033493;NM_033489 ;NM_033486;NM_033488;NM_033492;N M_033487 | Body;Body;Body;Body;Body;Body; 5'UTR | chr1:1602391-1602686 |
| cg18267374 | NEFM;NEFM;NEFM | NM_001105541;NM_005382;NM_005382 | TSS1500:5'UTR:1 stExon | chr8:24770908-24772547 |
| cg06894032 | SMTN;SMTN;SMTN | NM_006932;NM_134270;NM_134269 | TSS1500;TSS1500;TSS1500 | chr22:31476872-31477505 |
| cg12008047 | GDF15 | NM_004864 | 1stExon | chr19:18497063-18497269 |
| cg10993442 | RUNX3;RUNX3 | NM_004350;NM_001031680 | TSS1500;Body | chr1:25255527-25259005 |
| cg02571816 | PPP1R14A | NM_033256 | TSS1500 | chr19:38746638-38747379 |
| cg07949597 | ZNF75A | NM_153028 | TSS1500 | chrl 6:3355020-3356012 |
| cg19510075 | C4orf41 ;RWDD4A;C4orf41 ;C4orf4 1 ;C4orf41 | NM_199053;NM_152682;NM_021942;NM _021942;NM_199053 | 1 stExon;TSS200;1 stExon;5'UTR; 5'UTR | chr4:184579921-184580644 |
| cg11668923 | ADAM12;ADAM12;ADAM12;ADAM 12 | NM_003474;NM_021641;NM_003474;NM _021641 | 1 stExon;5'UTR;5'UTR;1 stExon | chr10:128076155-128077482 |
| cg16219603 | PENK | NM_001135690 | TSS1500 | chrB:57360585-57360815 |
| cg27262412 | TBX15 | NM_152380 | 5'UTR | chr1:119529819-119530712 |
| cg21924449 | GREM1 | NM_013372 | 5'UTR | chr15:33009530-33011696 |
| cg06361057 | KIAA0895L;EXOC3L | NM_001040715;NM_178516 | TSS1500;Body | chr16:67217716-67219219 |
| cg07382454 | TM6SF2 | NM_001001524 | 1 stExon | chr19:19383703-19384283 |
| cg08307171 | CSNK1G2 | NM_001319 | 5'UTR | chr19:1957774-1958038 |
| cg05144345 | FAM53A | NM_001013622 | TSS1500 | chr4:1685405-1686743 |
| cg12204732 | DOCK1 ;FAM1 96A | NM_001380;NM_001039762 | Body;TSS200 | chr10:128993505-128995167 |
| cg26273005 | WDR81;WDR81 ;WDR81 ;WDR81 | NM_001163673;NM_001163809; NM_001163811;NM_152348 | Body;1 stExon;5'UTR;5'UTR | chr17:1627745-1629897 |

H

| CpG site ID | Reference gene name | RefSeq mRNA accession number | UCSC Genome Browser gene category | CpG island location |
|---|---|---|---|---|
| cg12176783 | TCEA2;TCEA2 | NM 003195;NM_198723 | TSS200;5'UTR | chr20:62693942-62695282 |
| cg22092811 | C3orf26;FILIP1L;MIR548G;FILIP1 L;FILIP1L | NM 032359;NM_182909;NR 031662;NM _014890;NM_001042459 | Body;Body;Body;TSS200;Body | chr3:99594969-99595215 |
| cg10367214 | CCDC28A | NM_015439 | TSS200 | chr6:139094449-139095009 |
| cg19908812 | NPY1R | NM_000909 | 5'UTR | chr4:164252954-164253471 |
| cg26844077 | KIAA0182;KIAA0182 | NM_001134473;NM_014615 | 5'UTR;Body | chr16:85658742-85658998 |
| cg10943359 | MARCH1 | NM_001166373 | TSS200 | chr4:165304328-165305177 |
| cg05957603 | XPNPEP3;ST13 | NM_022098;NM_003932 | TSS1500;TSS200 | chr22:41 252146-41 253240 |
| cg27174972 | FAM5B | NM_021165 | 5'UTR | chr1:177150814-177151165 |
| cg01108476 | TCP11;TCP11 | NM_001093728;NM_018679 | TSS200;TSS200 | chr6:35108801-35109499 |
| cg00030420 | SLC25A2 | NM_031947 | TSS200 | chr5:140683195-140683773 |
| cg18301583 | ZNF415;ZNF415;ZNF415;ZNF415 | NM_001164309;NR_028343;NM_001136 038;NM_018355 | 5'UTR;Body;5'UTR;5'UTR | chr19:53635625-53636230 |
| cg05336395 | PCDHS;PCDHS | NM_002590;NM_032949 | 1 stExon;1 stExon | chr13:5341 9897-53422872 |
| cg06555246 | TIMM44;CTXN1 | NM_006351;NM_206833 | 3'UTR;TSS1500 | chr19:7991892-7992179 |
| cg24743682 | HIST1H3D;HIST1H2BF;HIST1H2A D | NM_003530;NM_003522;NM_021065 | TSS1500;1 stExon;TSS1 500 | chr6:26199838-26200120 |
| cg01963258 | RBMXL3;LRCH2 | NM_001145346;NM_020871 | 1 stExon;Body | chrX:114423906-114427195 |
| cg05465955 | COASY;COASY;COASY;COASY;C OASY | NM_025233;NM_001042529;NM_001042 532;NM_001042530NM_001042531 | 1 stExon;Body;Body;Body;5'UTR | chr17:40714087-40715282 |
| cg10372921 | LOXL1 | NM_005576 | TSS200 | chr15:74218696-74220373 |
| cg03738352 | SLC34A2 | NM 006424 | TSS200 | chr4:25657119-25657547 |
| cg21957174 | RASGRP2;RASGRP2;RASGRP2;R ASGRP2 | NM_001098671 ;NM_153819;NM_001098 670;NM_001098671 | 5'UTR;5'UTR;5'UTR;1 stExon | chr11:64509433-64513826 |
| cg17785250 | PCDHB7 | NM_018940 | 1 stExon | chr5:140553681-140554637 |
| cg19768599 | SLC12A8 | NM_024628 | TSS200 | chr3:124931161-124931747 |
| cg08677535 | KANK1 | NM_015158 | 5'UTR | chr9:503936-505722 |
| cg06532379 | ALPK3 | NM_020778 | 1 stExon | chr15:85360192-85360574 |
| cg00221494 | FARP1;FARP1 | NM_005766;NM_001001715 | TSS1 500;TSS1 500 | chr13:98794398-98796241 |
| cg08095852 | WDR17;WDR17;WDR17;WDR17 | NM_181265;NM_181265;NM_170710;NM _170710 | 1 stExon;5'UTR;1 stExon;5'UTR | chr4:176986921-176987360 |
| cg23249369 | GNAS;GNAS;GNAS;GNASAS | NM_080425;NM001077490;NM016592 ;NR_002785 | TSS1500;TSS1500;3'UTR;TSS1 500 | chr20:57426729-57427047 |
| cg02723964 | SOLH | NM_005632 | 5'UTR | chr16:593299-593500 |

**[Table 3]**

| CpG site ID | Reference gene name | RefSeq mRNA accession number | UCSC Genome Browser gene category | CpG island location |
|---|---|---|---|---|
| cg14036868 | A TL2A TL2A TL2 | NM_001135673:NR_024191;NM_022374 | TSS200;TSS200;TSS200 | chr2:38603569-38604510 |
| cg20475035 | PCDHB10 | NM_018930 | 1 stExon | chr5:140573423-140574316 |
| cg20119148 | PDE4C | NM_000923 | 5'UTR | chr19:18343496-18344196 |
| cg20649716 | PCDHB10 | NM_018930 | 1 stExon | chr5:140573423-140574316 |
| cg15858239 | NKAIN3;NKAIN3 | NM_173688;NM_173688 | 1 stExon;5'UTR | chrB:63160598-63162064 |
| cg23160016 | GABRA2;GABRA2;GABRA2 | NM_001114175;NM_000807;NM_000807 | TSS1500;1 stExon;5'UTR | chr4:46391903-46392572 |
| cg01155497 | PCDHA2;PCDHA1;PCDHA1;PCDH A3;PCDHA4;PCDHA5;PCDHA5 | NM_018905;NM_031411;NM_01 8900;NM _018906;NM_018907;NM031501;NM_01 8908 | Body;Body;Body;Body;Body;1 stEx on;1 stExon | chr5:140202573-140203610 |
| cg20692569 | FZD9 | NM_003508 | 1 stExon | chr7:72847933-72850032 |
| cg14132834 | ATP5SL;ATP5SL;ATP5SL;ATP5S L;ATP5SL;ATP5SL;ATP5SL | NR_030765;NM_001167867;NM_001167 868;NM_001167870;NM_001167869;NM _001167871;NM_018035 | TSS200;TSS1500;TSS1500;TS S200;TSS200;TSS200;TSS200 | chr19:41945690-41945904 |
| cg01097725 | PCDHB4 | NM_018938 | 1 stExon | chr5:140502890-140503804 |
| cg03772909 | PRR23A | NM_001134659 | TSS1500 | chr3:138724325-138725446 |
| cg23661309 | ADC;ADC | NM_052998;NM_052998 | 1 stExon;5'UTR | chr1:33546524-33547243 |
| cg14470647 | ABCA1 | NM_005502 | 5'UTR | chr9:107689847-107690798 |
| cg08336641 | L3MBTL4 | NM_173464 | 5'UTR | chr18:6413959-6414873 |
| cg08928145 | TSSK6;TSSK6 | NM_032037;NM_032037 | 3'UTR;1 stExon | chr19:19624954-19627258 |
| cg22152229 | PCDHA6;PCDHA2;PCDHA1;PCDH A1;PCDHA6;PCDHA5;PCDHA3;PC DHA4;PCDHA6 | NM_031849;NM_018905;NM_031411;NM _018900;NM_031848;NM_018908;NM_01 8906;NM_018907;NM_018909 | 1 stExon;Body;Body;Body;1 stExo n;Body;Body;Body;1 stExon | chr5:140208753-140209957 |
| cg13990746 | ANKRD45 | NM_198493 | TSS200 | chr1:173638662-173639045 |
| cg06656994 | FAM163A | NM_173509 | 5'UTR | chr1:179711947-179713951 |
| cg03474219 | ESRRA | NM_004451 | TSS1500 | chr11:64072064-64073914 |
| cg06188545 | PEAR1 | NM_001080471 | 5'UTR | chr1:156863415-156863711 |
| cg24670552 | IRS2 | NM_003749 | 1 stExon | chr13:110434466-110440180 |
| cg26027669 | GFI1 ;GFI1 | NM_001127216;NM_005263 | TSS1 500;TSS200 | chr1 :92945907-92952609 |
| cg16547027 | KCTD1;KCTD1;KCTD1 | NM_001136205;NM_198991;NM_001142 730 | 5'UTR;5'UTR;1 stExon | chr18:24126780-24131138 |
| cg07330212 | PCDHA2;PCDHA1;PCDHA1;PCDH A3;PCDHA4;PCDHA5;PCDHA5 | NM_018905;NM_031411;NM_01 8900;NM _018906;NM_018907;NM_031501;NM_01 8908 | Body;Body;Body;Body;Body;1 stEx on;1 stExon | chr5:140202573-140203610 |
| cg09370867 | FGR;FGR | NM_005248;NM_005248 | 1 stExon;5'UTR | chr1:27961 559-27961 810 |

**[Table 4]**

| CpG site ID | Reference gene name | RefSeq mRNA accession number | UCSC Genome Browser gene category | CpG island location |
|---|---|---|---|---|
| cg13332925 | LOC1 00130691 ;AGPS | NR_026966;NM_003659 | TSS200;1 stExon | chr2:178257227-178257892 |
| cg07221204 | CSNK1G1 | NM_022048 | TSS200 | chr15:64647914-64648523 |
| cg18957484 | APAF1;IKBIP;IKBIP;IKBIP;APAF1; APAF1;IKBIP;APAF1;IKBIP;APAF 1;IKBIP | NM-181868;NM_201612;NM_201612;NM _153687;NM_001160;NM_181869;NM_20 1613;NM_181861;NM_153687;NM _0132 29;NM_201613 | TSS1500;1 stExon;5'UTR;1 stExo n;TSS1500;TSS1500;5'UTR;TSS 1500;5'UTR;TSS1500'1 stExon | chr12:99038272-99039483 |
| cg03590031 | PRIC285;PRIC285 | NM_001037335;NM_033405 | Body;TSS1500 | chr20:62200085-62200320 |
| cg04868262 | ZBTB7B | NM_015872 | TSS1500 | chr1:154973250-154974358 |
| cg20180585 | TEF;TEF | NM_001145398;NM_003216 | Body;TSS1500 | chr22:41777430-41778281 |
| cg08467108 | EGLN2;EGLN2 | NM_080732;NM_053046 | TSS200;TSS1 500 | chr1 9:41 304467-41 305050 |
| cg05919661 | LMBR1L | NM_018113 | 1 stExon | chr12:49504035-49504642 |
| cg07569069 | C1 9orf39 | NM_175871 | TSS200 | chr19:11485286-11485769 |
| cg20494108 | MEA1;KLHDC3 | NM_014623;NM_057161 | TSS200;TSS1 500 | chr6:42981408-42982077 |
| cg25255679 | RHPN1 ;C8orf51 | NM_052924;NR_026785 | TSS200;TSS200 | chr8:144450376-144451557 |
| cg21735093 | FLCN;FLCN | NM_144997;NM_144606 | TSS200;TSS200 | chr17:17140117-17141080 |
| cg09441152 | PQLC1;PQLC1;PQLC1 | NM_025078;NM_001146343;NM_001146 345 | TSS1 500;TSS1 500;TSS1 500 | chr18:77710769-77712454 |
| cg26900259 | TRIM23;C5orf44;TRIM23;TRIM23; C5orf44;C5orf44;C5orf44 | NM_001656;NM_024941;NM_033228;NM _033227;NR_003545;NM_001093755;N M_001093756 | TSS200;TSS1 500;TSS200;TSS 200;TSS1500;TSS1500;TSS150 0 | chr5:6491 9914-64920288 |
| cg01115099 | PRR3;GNL1;PRR3;GNL1 | NM_025263;NM_005275;NM_001077497 ;NM_005275 | 1 stExon;5'UTR;1 stExon;1 stExon | chr6:30523775-30525189 |
| cg08228715 | PYCR1;PYCR1 | NM_153824;NM_006907 | TSS1500;TSS1500 | chr17:79894668-79895533 |
| cg19653594 | SPTBN4;BLVRB | NM_020971 ;NM_000713 | TSS1500;TSS1500 | chr19:40971476-40973393 |
| cg23422263 | BAZ1 A;BAZ1 A;BAZ1 A;BAZ1 A | NM_182648;NM_1 82648;NM_013448;NM _013448 | 1 stExon;5'UTR;1 stExon;5'UTR | chr14:35342845-35344526 |
| cg07786668 | ZFHX3 | NM₋001 164766 | 5'UTR | chr16:73091514-73092395 |
| cg16699331 | SLC25A46 | NM 138773 | TSS200 | chr5:110074605-110075223 |
| cg18130076 | RBM14 | NM_006328 | TSS200 | chr11:66383571-66385047 |
| cg18079631 | HNRNPC;HNRNPC;HNRNPC;HNR NPC | NM_001077443;NM_001077442;NM_031 314;NM_004500 | TSS200;TSS200;TSS200;TSS2 00 | chr14:21737406-21738094 |
| cg11472786 | HDAC11;HDAC11 | NM_001136041;NM_024827 | 5'UTR;Body | chr3:13521506-13522262 |
| cg03705718 | SLC16A14;SLC16A14 | NM_152527;NM_152527 | 1 stExon;5'UTR | chr2:230932468-230933550 |

| CpG site ID | Reference gene name | RefSeq mRNA accession number | UCSC Genome Browser gene category | CpG island location |
|---|---|---|---|---|
| cg15371777 | C4orf33;SCLT1;SCLT1 | NM_173487;NM_144643;NM_144643 | TSS200;5'UTR;1 stExon | chr4:130014349-130014899 |
| cg21700166 | GPR68 | NM_003485 | TSS200 | chr14:91719576-91720228 |
| cg19694491 | HSPA8;HSPA8 | NM_153201;NM_006597 | TSS200TSS200 | chr11:122932173-122933803 |
| cg02957620 | ZSWIM7;TTC19;ZSWIM7 | NM_001042698;NM_017775;NM_001042 697 | TSS1500;1 stExon;TSS1500 | chr17:15902500-15903688 |
| cg00450930 | PRPF40B | NM_012272 | TSS1500 | chr12:50016973-50017466 |
| cg03839341 | RAB10 | NM_016131 | TSS1500 | chr2:26256301-26257567 |
| cg09546172 | SLC37A1 | NM_018964 | 5'UTR | chr21:43933589-43934832 |
| cg15185106 | AMD1;AMD1 | NM_001033059;NM_001634 | TSS1500;TSS1500 | chr6:111195383-111197576 |
| cg16618634 | ANKLE2 | NM_015114 | TSS1500 | chr12:133337981-133338989 |
| cg06792598 | CD55;CD55 | NM_001114752;NM_000574 | TSS200TSS200 | chr1 :207494543-207495364 |
| cg09516349 | SSBP4;SSBP4 | NM_032627;NM_001009998 | TSS1500;TSS1500 | chr19:18527651-18531118 |
| cg16419136 | MGC23284;SNAI3 | NR_024399;NM_178310 | Body;1 stExon | chr16:88752520-88753278 |
| cg27302229 | SMG1 | NM_015092 | TSS1500 | chr16:18937030-18938126 |
| cg06089712 | BAZ1 A;BAZ1 A | NM_182648;NM_013448 | 5'UTR;5'UTR | chr14:35342845-35344526 |
| cg06535993 | APBB1IP | NM_019043 | TSS200 | chr10:26727061-26727992 |
| cg15456551 | NDUFB1;CPSF2 | NM_004545;NM_017437 | TSS200;TSS200 | chr14:92587549-92589022 |
| cg27353579 | MBTD1;UTP18 | NM_017643;NM_016001 | TSS1500;TSS200 | chr17:49337102-49338583 |
| cg04137594 | NPBWR2 | NM_005286 | 1 stExon | chr20:62737382-62738071 |
| cg05877850 | RWDD2A;PGM3;PGM3 | NM_033411;NM_015599;NM_01 5599 | TSS200;5'UTR;1 stExon | chr6:83902606-83903777 |
| cg18062360 | MZF1;MZF1;MZF1;LOC100131691 | NM_198055;NM_198055;NM_003422;NR _027334 | 5'UTR;1 stExon;5'UTR;Body | chr19:59084201-59085059 |
| cg20643362 | C19orf12;C1 9orf1 2;C19orf12 | NM_031448;NM_031448;NM_001031726 | 1 stExon;5'UTR;TSS1 500 | chr1 9:30206040-30206497 |
| cg26516363 | LIN54 | NM_001115007 | TSS1500 | chr4:83933332-83934370 |
| cg26013357 | PHLDA2 | NM_003311 | TSS1500 | chr11:2949788-2951756 |
| cg13997901 | SERINC5 | NM_178276 | TSS200 | chr5:79551369-79552294 |
| cg07730149 | MPV17L2 | NM_032683 | TSS1500 | chr19:18303623-18304425 |
| cg25577680 | ZNF10 | NM_015394 | 5'UTR | chr12:133706528-133707594 |
| cg01853632 | C3orf39;C3orf39 | NM_032806;NM_032806 | 5'UTR;1 stExon | chr3:43147184-43147713 |
| cg16429819 | GPX1;GPX1 | NM_000581;NM_201397 | 1 stExon;1 stExon | chr3:49394855-49395942 |
| cg11277126 | TRPC4AP;TRPC4AP | NM_199368;NM _015638 | TSS1500;TSS1500 | chr20:33680438-33681029 |
| cg19490297 | REST | NM_005612 | TSS200 | chr4:57773867-57775602 |
| cg01822573 | TBKBP1 | NM_014726 | TSS200 | chr17:45771107-45772993 |

The genes in the biological samples referred to in the foregoing are listed in Table 1-5. In the method of the present embodiment, the percentage of DNA methylation in CpG regions (e.g., CpG islands) of the promoter regions, untranslated regions or translated regions of these genes is determined. This is done by measuring the percentage methylation of cytosine residues in CpG regions.

Currently, the DNA sequences of genes included in the human genome may be easily obtained by searching gene names or registration numbers (reference numbers) in various databases that have been created. The aim of the present invention is to measure the percentage of DNA methylation in targeted CpG regions. With it, the percentage DNA methylation in CpG regions may be detected through a method that involves distinguishing between sequences with methylated cytosine residues and sequences without methylated cytosine residues in CpG regions.

A suitable method for detecting methylated cytosine residues uses bisulfite treatment of DNA fragments. In bisulfite treatment, cytosine residues are deaminated and converted to uracil residues, but if methylated, cytosine residues remain unchanged. A commercially available kit, such as EZ DNA Methylation Kit (Zymo Research), may be used for bisulfite treatment.

After bisulfite treatment, the methylation of cytosine residues can be detected by sequencing. The percentage of methylated cytosine residues can be measured by determining the base sequences of all DNA fragments using a next generation sequencer.

Alternatively, with DNA fragments after bisulfite treatment as templates, methylation of cytosine residues can be detected by means of a PCR method using primers to distinguish between cytosine residue and uracil residues. The percentage of methylated cytosine residues may be measured by performing quantitative PCR and creating a highresolution melting curve based on the reaction temperature for PCR products.

Another method is as follows. A probe capable of hybridization to the target region is designed. After bisulfite treatment, DNA fragments are subjected to whole genome amplification and fragmented, both processes using enzymes, hybridized to beads to which the probe is attached and fluorescently labeled nucleotides attached by a single nucleotide extension reaction. The percentage of methylated cytosine residues can then be measured by measuring the fluorescence intensity using fluorescent dye-labeled antibodies. This procedure can be conducted using a commercially available kit; for instance, Illumina Infinitum HD Methylation Assay Kit (Illumina), with bead analysis by means of iScan (Illumina).

The percentage of methylated cytosine resides may also be measured as follows. With DNA fragments following bisulfite treatment as templates, PCR amplification of target regions is carried out and then transcription into RNA. Next using RNA-ase, basespecific fragmentation is performed and methylated and unmethylated DNA fragments detected from their different molecular weights using a time-of-flight mass spectrometer. The percentage methylation may be measured using a commercially-available analysis kit; for instance, Mass ARRAY Epi-TYPER (SEQUENOM).

Yet another alternative is a PCR method using DNA fragments following bisulfite treatment as templates, and primers that distinguish between cytosine and uracil residues. By this method, the percentage methylation of cytosine residues is measured by means of quantitative PCR using probes labelled with 2 fluorescent dyes that allow cytosine and uracil residues to be distinguished.

### [Kit]

In one embodiment, this invention provides a kit to determine the likelihood that subjects will develop Alzheimer's disease, that includes primers or probes for the amplification of DNA methylation regions in CpG regions of promoter regions, untranslated regions or translated regions of at least one gene selected from groups consisting of the following: ELOVL2, OLIG3, SLC7A14, ACTA1, SOX17, DPYS, LOC728661, CDK11B, NEFM, SMTN, GDF15, RUNX3, PPP1R14A, ZNF75A, C4orf41, RWDD4A, ADAM12, PENK, TBX15, GREM1, KIAA0895L, EXOC3L, TM6SF2, CSNK1G2, FAM53A, DOCK1, FAM196A, WDR81, TCEA2, C3orf26, FILIP1L, MIR548G, CCDC28A, NPY1R, KIAA0182, MARCH1, XPNPEP3, ST13, FAM5B, TCP11, SLC25A2, ZNF415, PCDH8, TIMM44, CTXN1, HIST1H3D, HIST1H2BF, HIST1H2AD, RBMXL3, LRCH2, LOXL1, SLC34A2, RASGRP2, PCDHB7, SLC12A8, KANK1, ALPK3, FARP1, WDR17, GNAS, GNASAS, SOLH, ATL2, PCDHB10, PDE4C, NKAIN3, GABRA2, PCDHA2, PCDHA1, PCDHA3, PCDHA4, PCDHA5, FZD9, ATP5SL, PCDHB4, PRR23A, ADC, ABCA1, L3MBTL4, TSSK6, PCDHA6, ANKRD45, FAM163A, ESRRA, PEAR1, IRS2, GFI1, KCTD1, FGR, LOC100130691, AGPS, CSNK1G1, APAF1, IKBIP, PRIC285, ZBTB7B, TEF, EGLN2, LMBR1L, C19orf39, MEA1, KLHDC3, RHPN1, C8orf51, FLCN, PQLC1, TRIM23, C5orf44, PRR3, GNL1, PYCR1, SPTBN4, BLURB, BAZ1A, ZFHX3, SLC25A46, RBM14, HNRNPC, HDAC11, SLC16A14, C4orf33, SCLT1, GPR68, HSPA8, ZSWIM7, TTC19, PRPF40B, RAB10, SLC37A1, AMD1, ANKLE2, CD55, SSBP4, MGC23284, SNAI3, SMG1, APBB1IP, NDUFB1, CPSF2, MBTD1, UTP18, NPBWR2, RWDD2A, PGM3, MZF1, LOC100131691, C19orf12, LIN54, PHLDA2, SERINC5, MPV17L2, ZNF10, C3orf39, GPX1, TRPC4AP, REST and TBKBP1.

With the kit of this embodiment, the percentage of DNA methylation in CpG regions of the promoter regions, untranslated regions or translated regions of the genes mentioned above can be measured.

The kit of the present embodiment may also include sodium bisulfite besides the primers and probes mentioned above. Furthermore, it may also include various reagents for DNA extraction, hybridization and ligation and PCR reactions, as well as restriction enzymes.

### [Other embodiments]

In 1 embodiment, this invention provides for treatment of AD that includes measurement of the percentage of methylation in biological samples from subjects in CpG regions of promoter regions, untranslated regions or translated regions of at least one gene selected from groups consisting of the following: ELOVL2, OLIG3, SLC7A14, ACTA1, SOX17, DPYS, LOC728661, CDK11B, NEFM, SMTN, GDF15, RUNX3, PPP1R14A, ZNF75A, C4orf41, RWDD4A, ADAM12, PENK, TBX15, GREM1, KIAA0895L, EXOC3L, TM6SF2, CSNK1G2, FAM53A, DOCK1, FAM196A, WDR81, TCEA2, C3orf26, FILIP1L, MIR548G, CCDC28A, NPY1R, KIAA0182, MARCH1, XPNPEP3, ST13, FAM5B, TCP11, SLC25A2, ZNF415, PCDH8, TIMM44, CTXN1, HIST1H3D, HIST1H2BF, HIST1H2AD, RBMXL3, LRCH2, LOXL1, SLC34A2, RASGRP2, PCDHB7, SLC12A8, KANK1, ALPK3, FARP1, WDR17, GNAS, GNASAS, SOLH, ATL2, PCDHB 10, PDE4C, NKAIN3, GABRA2, PCDHA2, PCDHA1, PCDHA3, PCDHA4, PCDHA5, FZD9, ATP5SL, PCDHB4, PRR23A, ADC, ABCA1, L3MBTL4, TSSK6, PCDHA6, ANKRD45, FAM163A, ESRRA, PEAR1, IRS2, GFI1, KCTD1, FGR, LOC100130691, AGPS, CSNK1G1, APAF1, IKBIP, PRIC285, ZBTB7B, TEF, EGLN2, LMBR1L, C19orf39, MEA1, KLHDC3, RHPN1, C8orf51, FLCN, PQLC1, TRIM23, C5orf44, PRR3, GNL1, PYCR1, SPTBN4, BLURB, BAZ1A, ZFHX3, SLC25A46, RBM14, HNRNPC, HDAC11, SLC16A14, C4orf33, SCLT1, GPR68, HSPA8, ZSWIM7, TTC19, PRPF40B, RAB10, SLC37A1, AMD1, ANKLE2, CD55, SSBP4, MGC23284, SNAI3, SMG1, APBB1IP, NDUFB1, CPSF2, MBTD1, UTP18, NPBWR2, RWDD2A, PGM3, MZF1, LOC100131691, C19orf12, LIN54, PHLDA2, SERINC5, MPV17L2, ZNF10, C3orf39, GPX1, TRPC4AP, REST and TBKBP1, and offering treatment for AD when the percentage of methylation is higher or lower in subjects than in NCs.

In the treatment method of this embodiment, the controls, biological samples, and procedures for the measurement of DNA methylation in CpG regions are the same as those mentioned above.

Currently available treatments for Alzheimer's disease include drug therapy, such as that with Aricept (registered trademark) and Reminyl (registered trade mark), as well as non-drug therapy methods such as those targeting the patient's behavioral or psychological symptoms.

### EXAMPLES

The following example is illustrative of the present invention but its use is not limited to this specific example.

### [Example 1]

Using a EZ DNA Methylation Kit (Zymo Research), bisulfite treatment, DNA purification and recovery were performed for 0.5 -1.0 µg peripheral blood samples from 4 elderly NCs (no dementia), and 15 subjects with AD or aMCI, its earlier stage (AD: 11, aMCI: 4), as well as 10 NCs ranging in age from 26-62 years. After alkali degeneration of the bisulfited DNA, the whole genome was amplified using enzymes. The amplified genome DNA was then fragmented by means of enzyme treatment, purified through precipitation with isopropanol and resuspended in hybridization buffer. The re-suspended DNA was then heat denatured, applied to Human Methylation 450 BeadChip (Illumina) or Infinium Methylation EPIC BeadChip (Illumina) and hybridized for 23 hours in a hybridization oven. After hybridization, the BeadChip was washed with buffer and through a single nucleotide elongation reaction, a single base labeled nucleotide was incorporated at the end of the probe. Next, the BeadChip was stained using a fluorescent marker antibody against the incorporated labeled nucleotide. After washing, coating and drying the stained BeadChip, fluorescence images were obtained using iScan Control Software ver. 3.2.45 (Illumina).

Background Subtraction and normalization to Internal Controls was then performed using GenomeStudio ver. 2011.1/Methylation Module ver. 1.9.0 (Illumina, Inc.).

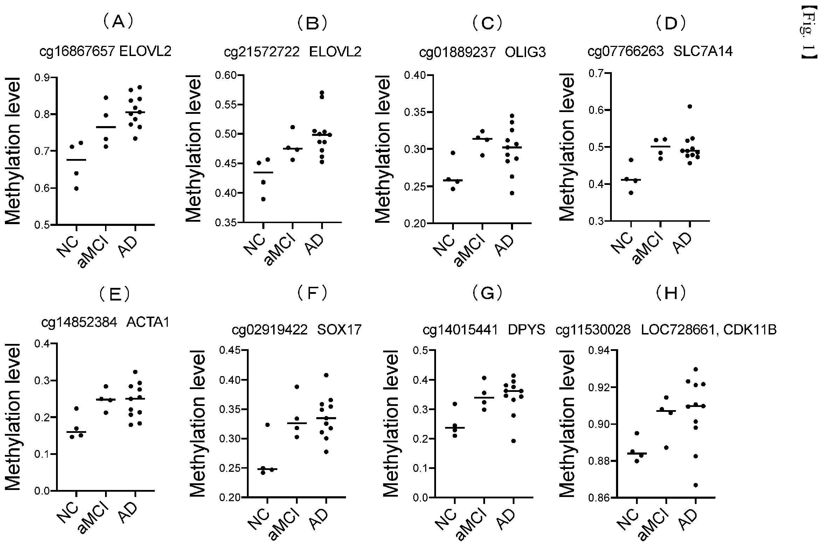

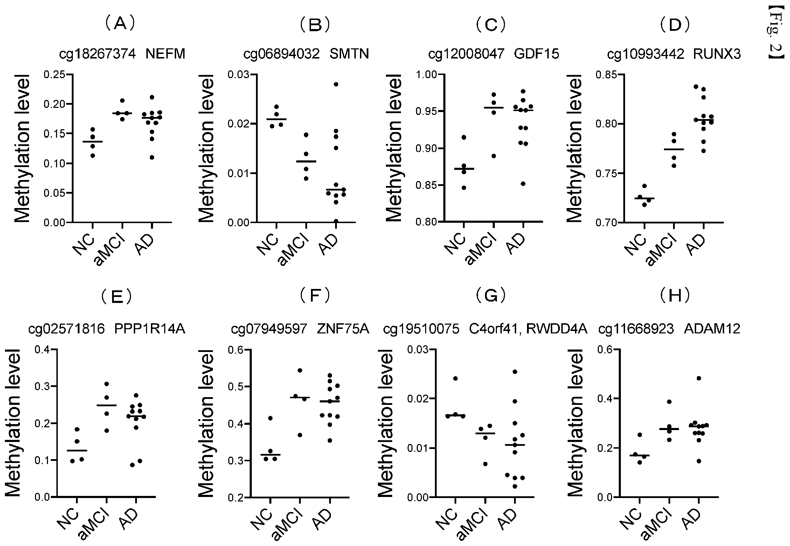

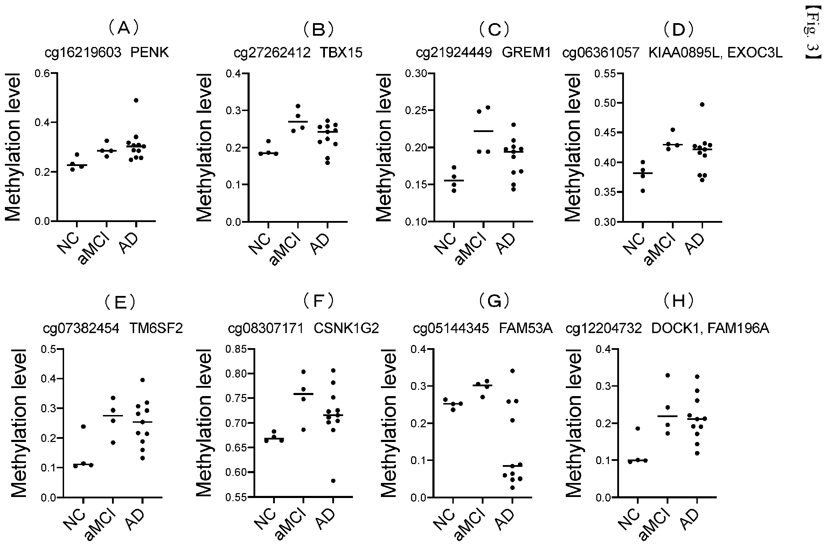

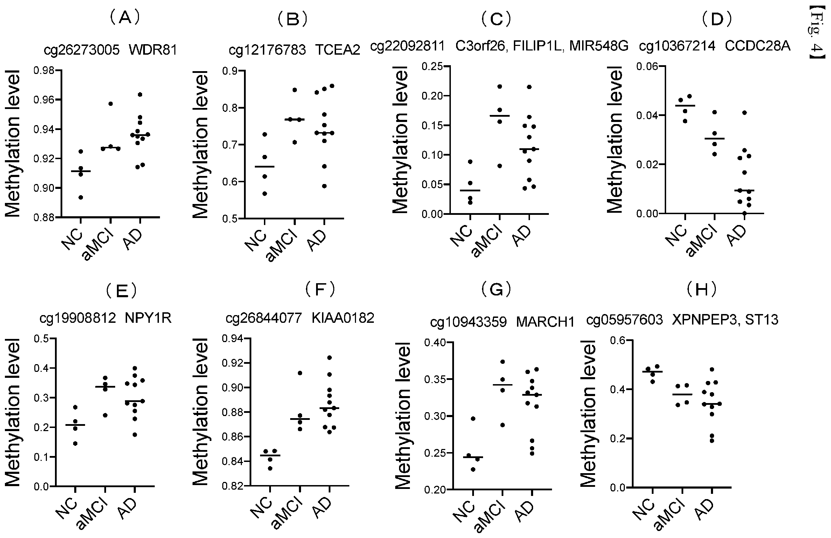

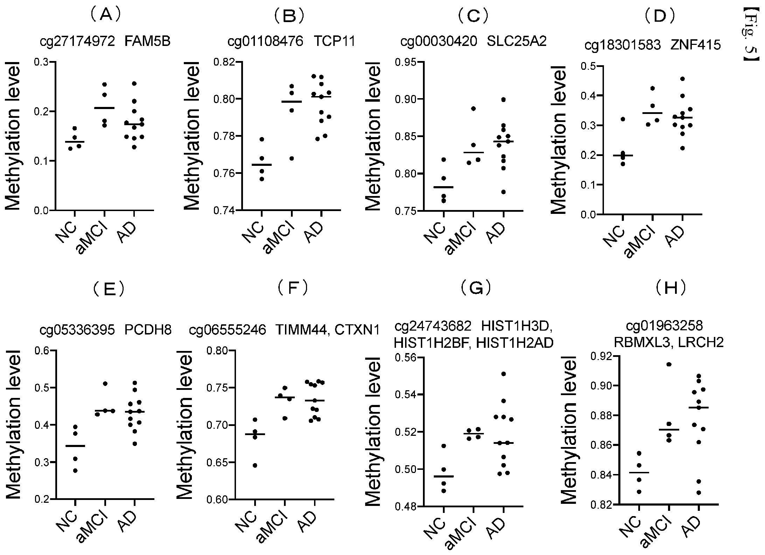

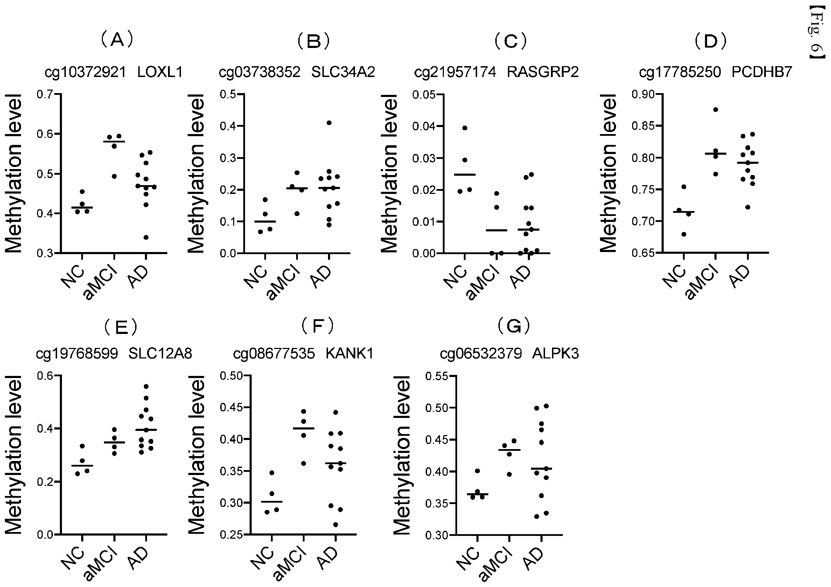

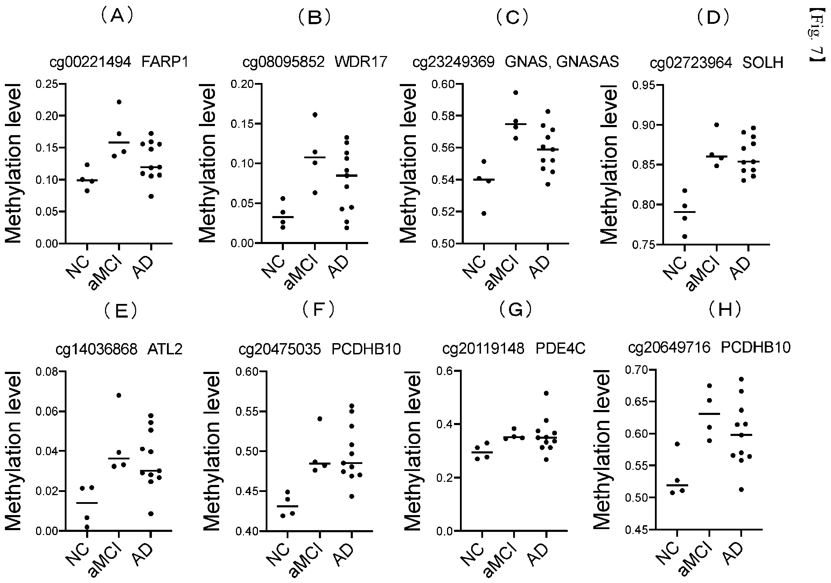

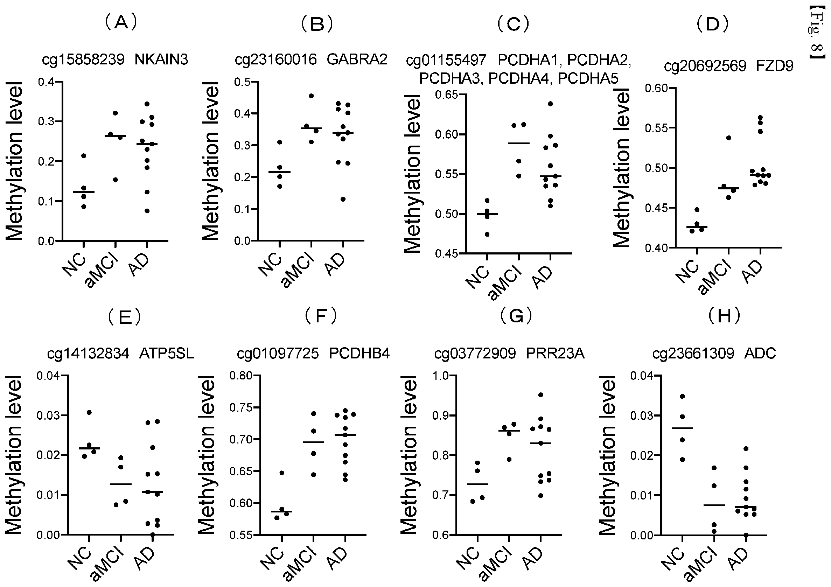

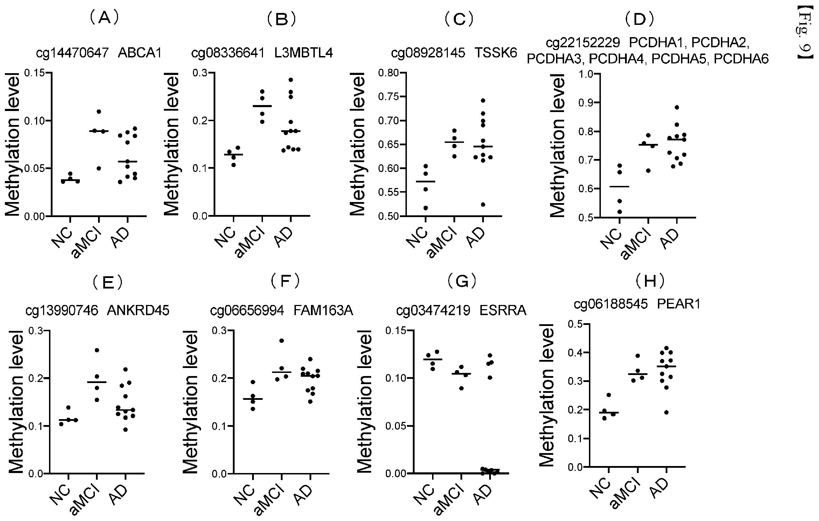

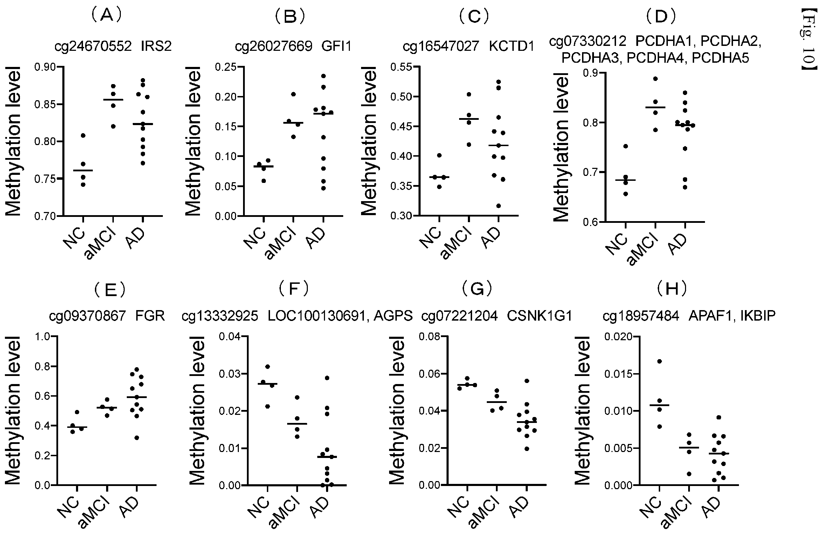

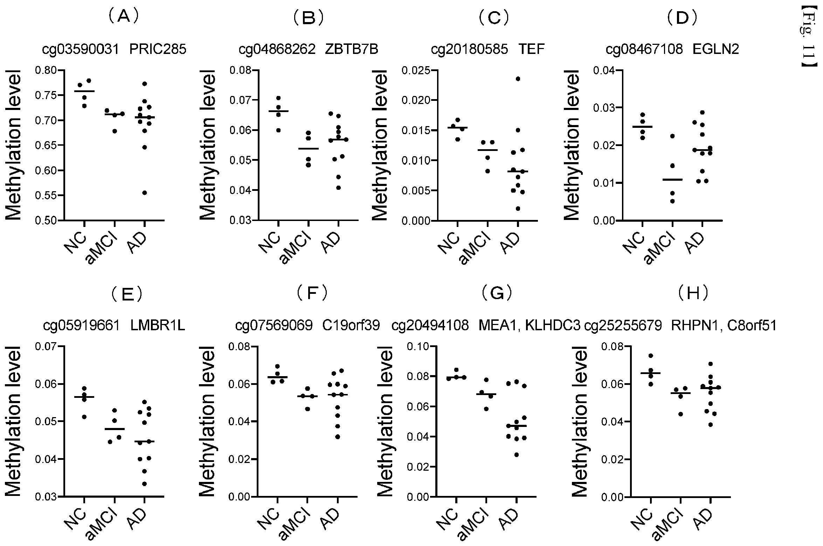

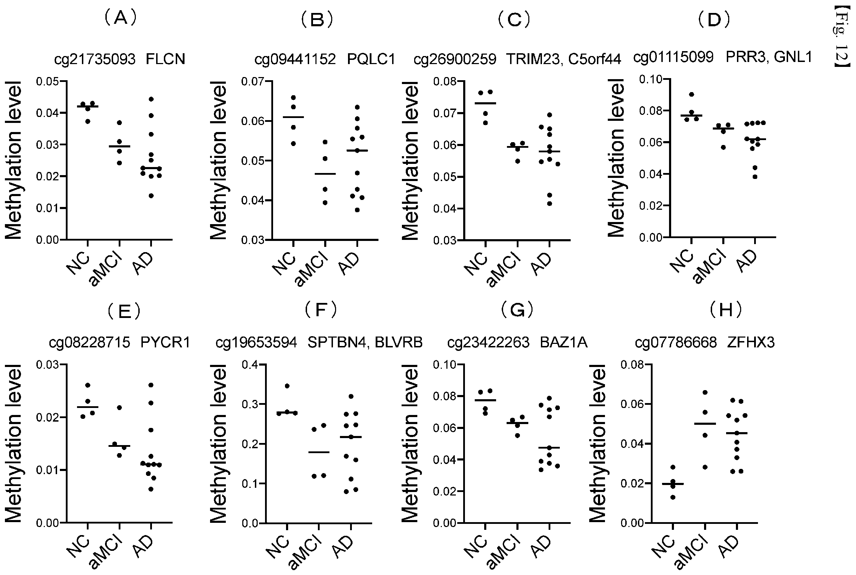

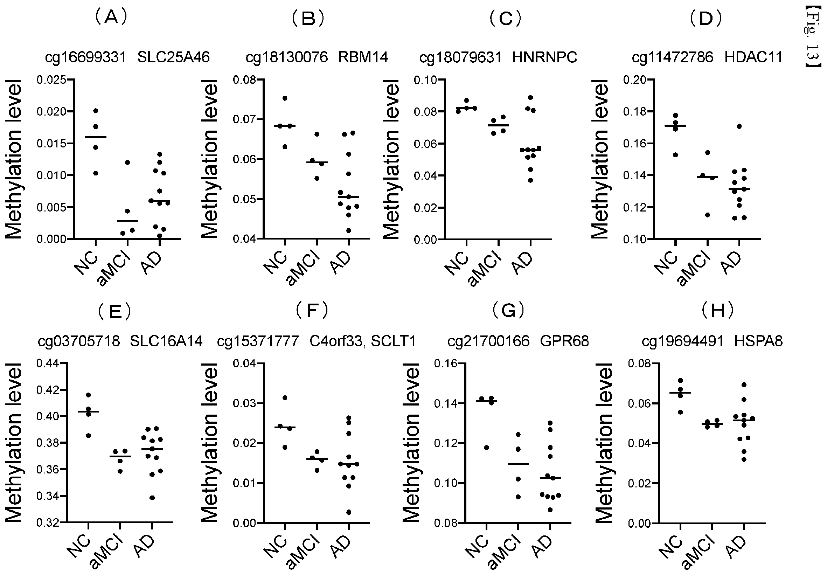

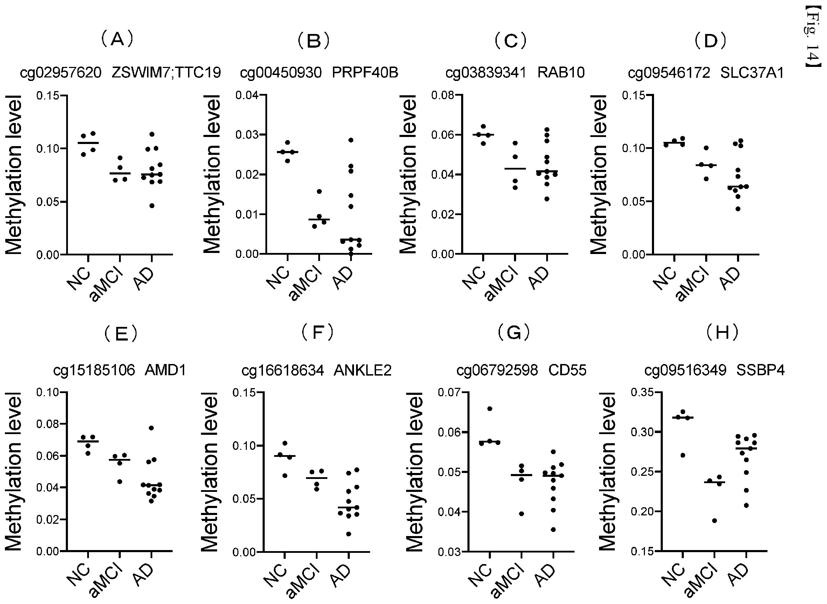

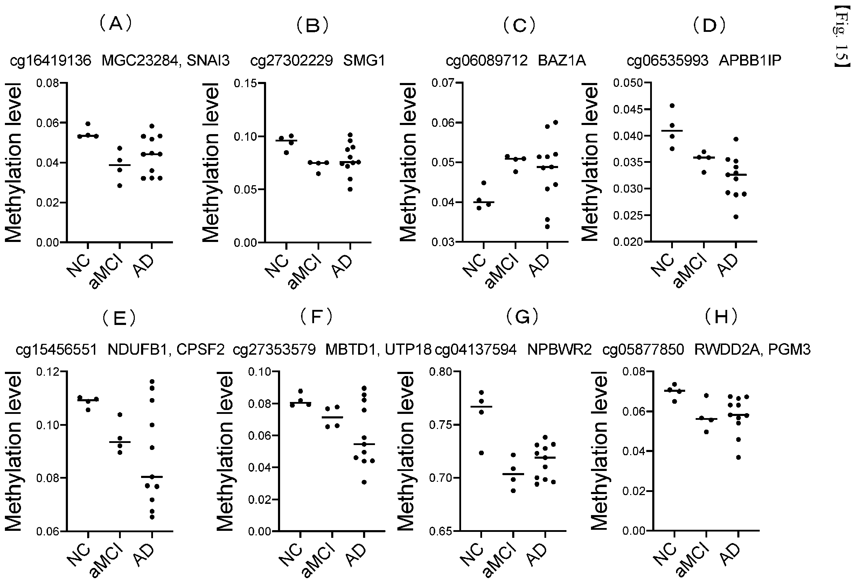

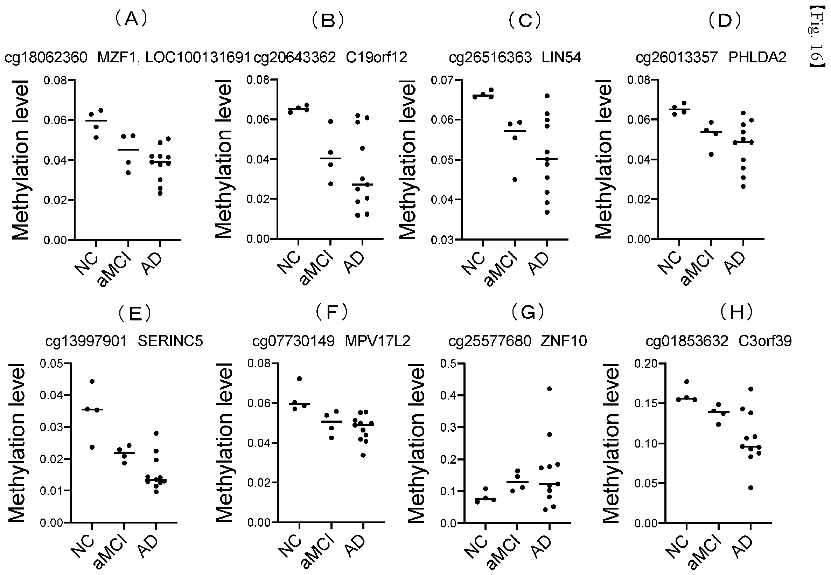

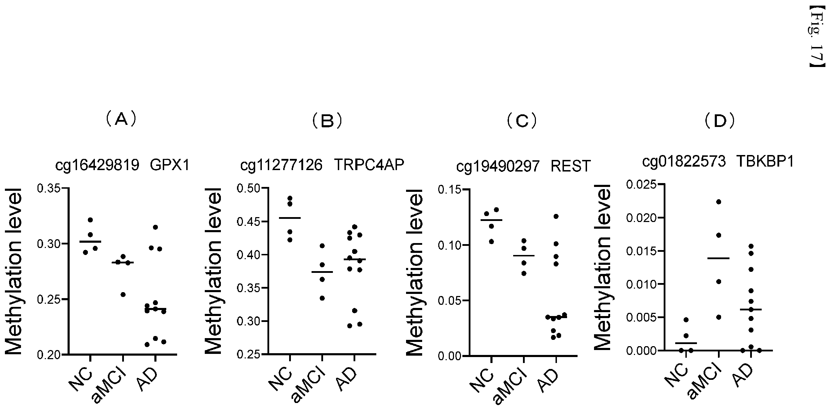

The DNA methylation analysis identified DNA methylation regions (DMRs) in which methylation was significantly higher or lower in AD and aMCI subjects than in NCs. These DMRs were common to those in which methylation was correlated with age. The results showed that the percentage of DNA methylation was significantly increased or reduced in CpG islands of promoter regions, untranslated regions or translated regions in the following genes: ELOVL2, OLIG3, SLC7A14, ACTA1, SOX17, DPYS, LOC728661, CDK11B, NEFM, SMTN, GDF15, RUNX3, PPP1R14A, ZNF75A, C4orf41, RWDD4A, ADAM12, PENK, TBX15, GREM1, KIAA0895L, EXOC3L, TM6SF2, CSNK1G2, FAM53A, DOCK1, FAM196A, WDR81, TCEA2, C3orf26, FILIP1L, MIR548G, CCDC28A, NPY1R, KIAA0182, MARCH1, XPNPEP3, ST13, FAM5B, TCP11, SLC25A2, ZNF415, PCDH8, TIMM44, CTXN1, HIST1H3D, HIST1H2BF, HIST1H2AD, RBMXL3, LRCH2, LOXL1, SLC34A2, RASGRP2, PCDHB7, SLC12A8, KANK1, ALPK3, FARP1, WDR17, GNAS, GNASAS, SOLH, ATL2, PCDHB10, PDE4C, NKAIN3, GABRA2, PCDHA2, PCDHA1, PCDHA3, PCDHA4, PCDHA5, FZD9, ATP5SL, PCDHB4, PRR23A, ADC, ABCA1, L3MBTL4, TSSK6, PCDHA6, ANKRD45, FAM163A, ESRRA, PEAR1, IRS2, GFI1, KCTD1, FGR, LOC100130691, AGPS, CSNK1G1, APAF1, IKBIP, PRIC285, ZBTB7B, TEF, EGLN2, LMBR1L, C19orf39, MEA1, KLHDC3, RHPN1, C8orf51, FLCN, PQLC1, TRIM23, C5orf44, PRR3, GNL1, PYCR1, SPTBN4, BLURB, BAZ1A, ZFHX3, SLC25A46, RBM14, HNRNPC, HDAC11, SLC16A14, C4orf33, SCLT1, GPR68, HSPA8, ZSWIM7, TTC19, PRPF40B, RAB10, SLC37A1, AMD1, ANKLE2, CD55, SSBP4, MGC23284, SNAI3, SMG1, APBB1IP, NDUFB1, CPSF2, MBTD1, UTP18, NPBWR2, RWDD2A, PGM3, MZF1, LOC100131691, C19orf12, LIN54, PHLDA2, SERINC5, MPV17L2, ZNF10, C3orf39, GPX1, TRPC4AP, REST and TBKBP1. Figures 1 - 17 show the methylation levels in CpG islands for individual genes for elderly NCs, AD patients and aMCI patients. Figures 1 - 17 also show methylation levels in the CpG islands of individual genes by Cg number. Owing to this, genes with the same name are shown on separate graphs if DNA methylation positions in CpG islands and the assigned Cg number differ. In addition, genes with different names are shown on the same graph if the DNA methylation position in the CpG island is the same and the assigned Cg number is the same.

As indicated in Figures 1-17, methylation levels in CpG islands of promoter regions, untranslated regions or translated regions in the following genes were significantly higher or lower in AD patients and aMCI patients as compared with NCs: ELOVL2, OLIG3, SLC7A14, ACTA1, SOX17, DPYS, LOC728661, CDK11B, NEFM, SMTN, GDF15, RUNX3, PPP1R14A, ZNF75A, C4orf41, RWDD4A, ADAM12, PENK, TBX15, GREM1, KIAA0895L, EXOC3L, TM6SF2, CSNK1G2, FAM53A, DOCK1, FAM196A, WDR81, TCEA2, C3orf26, FILIP1L, MIR548G, CCDC28A, NPY1R, KIAA0182, MARCH1, XPNPEP3, ST13, FAM5B, TCP11, SLC25A2, ZNF415, PCDH8, TIMM44, CTXN1, HIST1H3D, HIST1H2BF, HIST1H2AD, RBMXL3, LRCH2, LOXL1, SLC34A2, RASGRP2, PCDHB7, SLC12A8, KANK1, ALPK3, FARP1, WDR17, GNAS, GNASAS, SOLH, ATL2, PCDHB 10, PDE4C, NKAIN3, GABRA2, PCDHA2, PCDHA1, PCDHA3, PCDHA4, PCDHA5, FZD9, ATP5SL, PCDHB4, PRR23A, ADC, ABCA1, L3MBTL4, TSSK6, PCDHA6, ANKRD45, FAM163A, ESRRA, PEAR1, IRS2, GFI1, KCTD1, FGR, LOC100130691, AGPS, CSNK1G1, APAF1, IKBIP, PRIC285, ZBTB7B, TEF, EGLN2, LMBR1L, C19orf39, MEA1, KLHDC3, RHPN1, C8orf51, FLCN, PQLC1, TRIM23, C5orf44, PRR3, GNL1, PYCR1, SPTBN4, BLURB, BAZ1A, ZFHX3, SLC25A46, RBM14, HNRNPC, HDAC11, SLC16A14, C4orf33, SCLT1, GPR68, HSPA8, ZSWIM7, TTC19, PRPF40B, RAB10, SLC37A1, AMD1, ANKLE2, CD55, SSBP4, MGC23284, SNAI3, SMG1, APBB1IP, NDUFB1, CPSF2, MBTD1, UTP18, NPBWR2, RWDD2A, PGM3, MZF1, LOC100131691, C19orf12, LIN54, PHLDA2, SERINC5, MPV17L2, ZNF10, C3orf39, GPX1, TRPC4AP, REST and TBKBP1

It has been found that there is a strong likelihood of aMCI in patients progressing to AD. The above results indicate that the likelihood of developing AD can be determined by measuring the percentage of methylated DNA in CpG islands of promoter regions, untranslated regions or translated regions in these genes.

### Industrial Applicability

The present invention provides a technique for determining the likelihood of developing Alzheimer's disease.

## Claims

1. A data collection method for determining the likelihood that a subject will develop AD that includes measuring the percentage of DNA methylation in biological samples from subjects in CpG regions of promoter regions, untranslated regions or translated regions of at least one gene selected from groups consisting of the following, wherein higher or lower methylation levels compared with NCs indicate that a subject is at risk of developing AD: ELOVL2, OLIG3, SLC7A14, ACTA1, SOX17, DPYS, LOC728661, CDK11B, NEFM, SMTN, GDF15, RUNX3, PPP1R14A, ZNF75A, C4orf41, RWDD4A, ADAM12, PENK, TBX15, GREM1, KIAA0895L, EXOC3L, TM6SF2, CSNK1G2, FAM53A, DOCK1, FAM196A, WDR81, TCEA2, C3orf26, FILIP1L, MIR548G, CCDC28A, NPY1R, KIAA0182, MARCH1, XPNPEP3, ST13, FAM5B, TCP11, SLC25A2, ZNF415, PCDH8, TIMM44, CTXN1, HIST1H3D, HIST1H2BF, HIST1H2AD, RBMXL3, LRCH2, LOXL1, SLC34A2, RASGRP2, PCDHB7, SLC12A8, KANK1, ALPK3, FARP1, WDR17, GNAS, GNASAS, SOLH, ATL2, PCDHB10, PDE4C, NKAIN3, GABRA2, PCDHA2, PCDHA1, PCDHA3, PCDHA4, PCDHA5, FZD9, ATP5SL, PCDHB4, PRR23A, ADC, ABCA1, L3MBTL4, TSSK6, PCDHA6, ANKRD45, FAM163A, ESRRA, PEAR1, IRS2, GFI1, KCTD1, FGR, LOC100130691, AGPS, CSNK1G1, APAF1, IKBIP, PRIC285, ZBTB7B, TEF, EGLN2, LMBR1L, C19orf39, MEA1, KLHDC3, RHPN1, C8orf51, FLCN, PQLC1, TRIM23, C5orf44, PRR3, GNL1, PYCR1, SPTBN4, BLURB, BAZ1A, ZFHX3, SLC25A46, RBM14, HNRNPC, HDAC11, SLC16A14, C4orf33, SCLT1, GPR68, HSPA8, ZSWIM7, TTC19, PRPF40B, RAB10, SLC37A1, AMD1, ANKLE2, CD55, SSBP4, MGC23284, SNAI3, SMG1, APBB1IP, NDUFB1, CPSF2, MBTD1, UTP18, NPBWR2, RWDD2A, PGM3, MZF1, LOC100131691, C19orf12, LIN54, PHLDA2, SERINC5, MPV17L2, ZNF10, C3orf39, GPX1, TRPC4AP, REST and TBKBP1.

2. Date collection method indicated in Claim 1, wherein biological samples are blood.

3. Kit for determining the likelihood that a subject will develop AD that includes primers or probes for amplifying DNA methylation regions in CpG regions of promoter regions, untranslated regions or translated regions of at least one gene selected from groups consisting of the following: ELOVL2, OLIG3, SLC7A14, ACTA1, SOX17, DPYS, LOC728661, CDK11B, NEFM, SMTN, GDF15, RUNX3, PPP1R14A, ZNF75A, C4orf41, RWDD4A, ADAM12, PENK, TBX15, GREM1, KIAA0895L, EXOC3L, TM6SF2, CSNK1G2, FAM53A, DOCK1, FAM196A, WDR81, TCEA2, C3orf26, FILIP1L, MIR548G, CCDC28A, NPY1R, KIAA0182, MARCH1, XPNPEP3, ST13, FAM5B, TCP11, SLC25A2, ZNF415, PCDH8, TIMM44, CTXN1, HIST1H3D, HIST1H2BF, HIST1H2AD, RBMXL3, LRCH2, LOXL1, SLC34A2, RASGRP2, PCDHB7, SLC12A8, KANK1, ALPK3, FARP1, WDR17, GNAS, GNASAS, SOLH, ATL2, PCDHB10, PDE4C, NKAIN3, GABRA2, PCDHA2, PCDHA1, PCDHA3, PCDHA4, PCDHA5, FZD9, ATP5SL, PCDHB4, PRR23A, ADC, ABCA1, L3MBTL4, TSSK6, PCDHA6, ANKRD45, FAM163A, ESRRA, PEAR1, IRS2, GFI1, KCTD1, FGR, LOC100130691, AGPS, CSNK1G1, APAF1, IKBIP, PRIC285, ZBTB7B, TEF, EGLN2, LMBR1L, C19orf39, MEA1, KLHDC3, RHPN1, C8orf51, FLCN, PQLC1, TRIM23, C5orf44, PRR3, GNL1, PYCR1, SPTBN4, BLURB, BAZ1A, ZFHX3, SLC25A46, RBM14, HNRNPC, HDAC11, SLC16A14, C4orf33, SCLT1, GPR68, HSPA8, ZSWIM7, TTC19, PRPF40B, RAB10, SLC37A1, AMD1, ANKLE2, CD55, SSBP4, MGC23284, SNAI3, SMG1, APBB1IP, NDUFB1, CPSF2, MBTD1, UTP18, NPBWR2, RWDD2A, PGM3, MZF1, LOC100131691, C19orf12, LIN54, PHLDA2, SERINC5, MPV17L2, ZNF10, C3orf39, GPX1, TRPC4AP, REST and TBKBP1.
